# EUROPEAN PATENT APPLICATION

(11) **EP 2 260 810 A1**
(43) Date of publication of application: **15.12.2010**
(21) Application number: 09728464.0
(22) Date of filing: 20.02.2009
(51) Int. Cl.: A61F 13/15, A61F 13/472, A61F 13/53, A61F 13/534, A61F 13/539

(54) **ABSORPTIVE ARTICLE AND METHOD OF PRODUCING THE SAME**

(30) Priority: 31.03.2008 JP 2008092750
(71) Applicant: Unicharm Corporation, Ehime 799-0111 (JP)
(72) Inventor: HARADA, Hiroyuki, Kanonji-shi Kagawa 769-1602 (JP); TAMURA, Tatsuya, Kanonji-shi Kagawa 769-1602 (JP)
(74) Representative: Eke, Philippa Dianne
(86) International application number: PCT/JP2009/053612
(87) International publication number: WO 2009/122830

(57) **Abstract**

An absorbent article with high fitting property and high absorptivity, where the end part is less deformed, is provided.

An absorbent article comprising at least a liquid-permeable sheet, a liquid-impermeable sheet, and an absorber sandwiched between the liquid-permeable sheet and the liquid-impermeable sheet, wherein the absorber comprises a first absorbing part and a second absorbing part formed nearly on the center part of the first absorbing part and smaller in the area than the first absorbing part, and a compressed groove is continuously provided in nearly closed form to extend from the peripheral edge region in the longitudinal direction of the second absorbing part to the first absorbing part.

## Description

### TECHNICAL FIELD

The present invention relates to an absorbent article. More specifically, the present invention relates to an absorbent article having a compressed groove provided at a specific position of an absorber, such as sanitary napkin, sheet for urinary leakage, etc.

### BACKGROUND ART

Conventionally, the absorber for an absorbent article such as sanitary napkin, etc. consists of a mere stack of pulp fibers and therefore, has a problem that the absorbent article becomes flat or deteriorates in the fitting property during usage, or its absorption capacity is low.

As regards the absorbent article succeeded in solving such a problem, an absorbent article is disclosed in Japanese Unexamined Patent Publication (Kokai) No. 11-358, comprising a first pulp layer and a second pulp layer stacked thereon and the second pulp layer being smaller in the area than the first pulp layer, where a specific groove part is formed in the peripheral edge region of the second pulp layer, and wherein virtue of the formation of the groove part, the absorber has a three-level structure consisting of an absorbing part containing the first and second pulp layers inside of the groove part, an absorbing part containing the first and second pulp layers outside of the groove part but inside of the peripheral edge of the second pulp layer, and an absorbing part containing the first pulp layer outside of the peripheral edge of the second pulp layer. This absorbent article is particularly **characterized in that** the absorber and a surface sheet are partially integrated at the production of the absorber, and deformation due to twisting can be thereby prevented.

### DISCLOSURE OF THE INVENTION

However, in the absorbent article of Japanese Unexamined Patent Publication (Kokai) No. 11-358, a groove part is not present at all in the first pulp layer of the absorber, and therefore the absorbent article practically suffers from bad rigidity distribution and allows the end part of the absorbent article to readily deform due to twisting in the first pulp layer.

Accordingly, an object of the present invention is to provide an absorbent article with high fitting property and high absorptivity, where the end part of the absorbent article is free from deformation due to twisting in the first pulp layer.

Under these circumstances, the present inventors have made intensive studies and found that an absorber in which two absorbing parts differing in the area from each other are integrated, having a continuous compressed groove extending across those two absorbing parts, can solve the above-described object. The present invention has been accomplished based on this finding.

That is, (1) the present invention provides an absorbent article comprising at least a liquid-permeable sheet, a liquid-impermeable sheet, and an absorber sandwiched between the liquid-permeable sheet and the liquid-impermeable sheet,
wherein the absorber comprises a first absorbing part, and a second absorbing part formed nearly on the center part of the first absorbing part and the second absorbing part being smaller in the area than the first absorbing part, and
a compressed groove is continuously provided in nearly closed form to extend from the peripheral edge region in the longitudinal direction of the second absorbing part to the first absorbing part.
(2) The present invention provides the absorbent article according to (1), wherein the depth of the compressed groove continuously provided in the peripheral edge region in the longitudinal direction of the second absorbing part is a depth sufficient to penetrate the second absorbing part and reach the first absorbing part.
(3) The present invention provides the absorbent article according to (1) or (2), wherein the absorber has an intermittent portion excluded from formation of the compressed groove, in the peripheral end part in the longitudinal direction of the second absorbing part.
(4) The present invention provides the absorbent article according to (1) or (2), wherein the absorber has an intermittent portion excluded from formation of the compressed groove, in the peripheral end part in the longitudinal direction of the first absorbing part.
(5) The present invention provides the absorbent article according to any one of (1) to (4), which is housed in a package in a state of being folded in three by making the intermittent portion work as a fold line.
(6) The present invention provides a production method of an absorbent article, comprising stacking an absorber comprising a first absorbing part and a second absorbing part formed nearly on the center part of the first absorbing part and the second absorbing part being smaller in the area than the first absorbing part, a mount and a liquid-permeable sheet in this order on a mount, and continuously applying embossing from the liquid-permeable sheet side over a region from the peripheral edge region in the longitudinal direction of the second absorbing part to the first absorbing part, thereby providing a compressed groove extending from the second absorbing part to the first absorbing part.

In the absorbent article of the present invention, the absorber has a compressed groove extending across the first absorbing part and the second absorbing part, so that the absorbent article can have a gentle change of rigidity and can be reduced in the uncomfortable feeling during usage. Also, as compared with conventional products, deformation due to twisting in the first absorbing part can hardly occur, as a result, high fitting property-and high absorptivity can be achieved.

### Brief Description of the Drawings

Fig. 1 is a plan view of one embodiment of the absorbent article of the present invention.
Fig. 2 is a Y-Y' cross-sectional view of Fig. 1.
Fig. 3 is a plan view of a preferred embodiment of the absorbent article of the present invention.

### Best Mode for Carrying Out the Invention

The present invention is described below by referring to the drawings, but the present invention is not limited to those illustrated in the drawings.

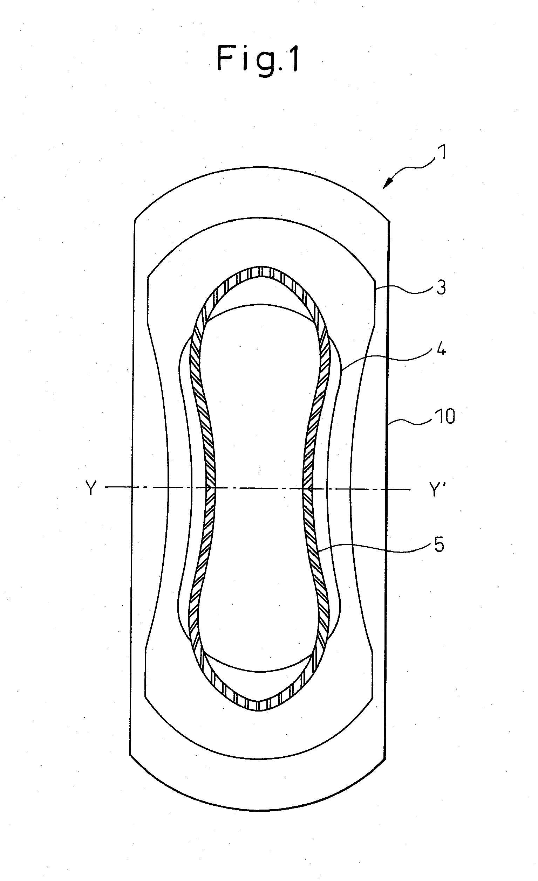

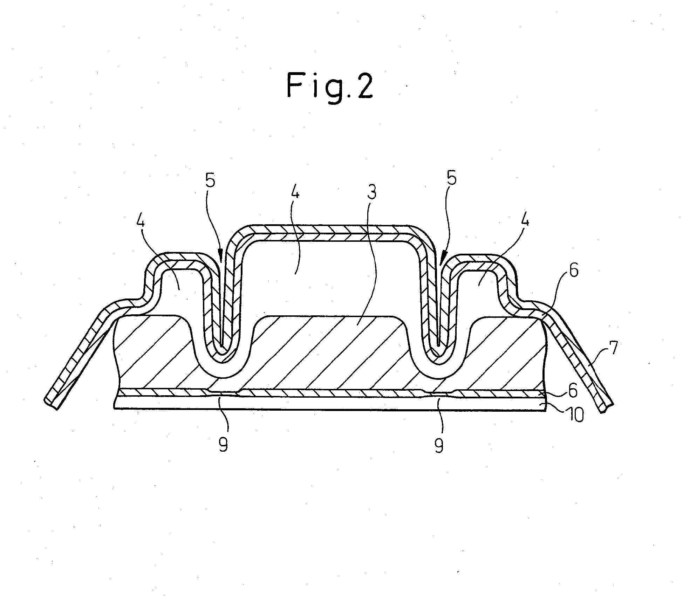

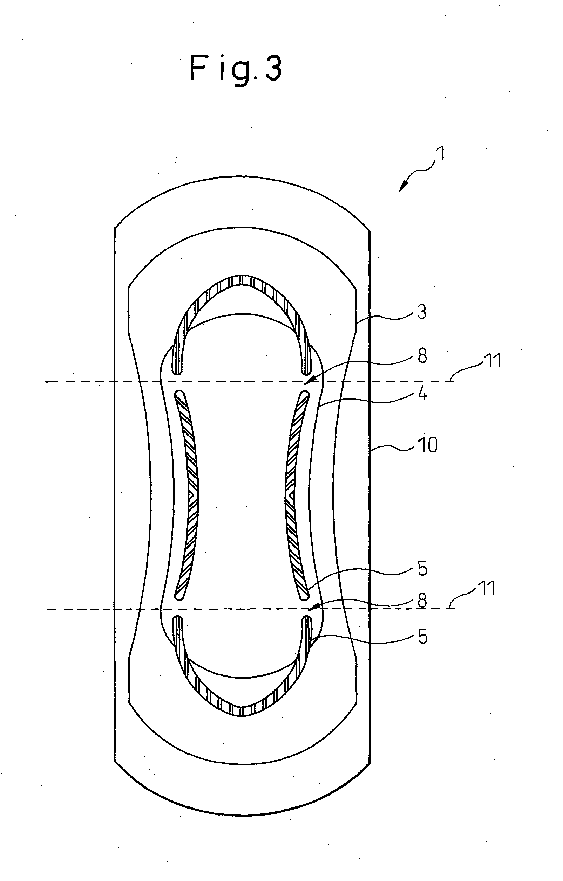

Fig. 1 is a plan view illustrating one embodiment of the absorbent article of the present invention, and Fig. 2 is its Y-Y' cross-sectional view. Also, Fig. 3 is a plan view illustrating a preferred embodiment of the absorbent article of the present invention.

The absorbent article 1 of the present invention comprises at least a liquid-permeable sheet 7, a liquid-impermeable sheet 10 and an absorber 2. The absorber is usually integrated with a mount 6, and the integrated absorber is sandwiched between the liquid-permeable sheet 7 and the liquid-impermeable sheet 10. As illustrated in Fig. 1, the absorber comprises a first absorbing part 3 and a second absorbing part 4, where the area of the second absorbing part is smaller than the area of the first absorbing part and the second absorbing part is integrated nearly on the center part of the first absorbing part. The shape of the absorber for use in the present invention is not particularly limited as long as it is a shape formed by integrating two absorbers differing in the area, and as illustrated in Fig. 2, there may take a configuration where a layer composed of a first absorber and a layer composed of a second absorber are previously stacked. That is, it may be sufficient if the portion in which a second absorbing part is disposed is higher in the basis weight than the portion where only a first absorbing part is present.

Although differing depending on usage or the like of the absorber, the thickness of the first absorbing part 3 is generally on the order of 1 to 10 mm, and the thickness of the second absorbing part 4 is generally on the order of 2 to 30 mm. Also, the basis weight of the first absorbing part 3 is generally on the order of 100 to 400 g/m², and the basis weight of the second absorbing part 4 is generally on the order of 200 to 1,200 g/m².

In the absorber, a compressed groove 5 is formed to extend from the peripheral edge region in the longitudinal direction of the second absorbing part to the first absorbing part. The compressed groove is continuously formed to take a nearly closed shape in planar view. For example, as illustrated in Fig. 1, the second absorbing part is shaped like a tongue, and the compressed groove is formed in the peripheral edge region in the longitudinal direction of the second absorbing part to draw an arching line curved with respect to the center of the absorbent article and is formed to further extend in a U-shape from the peripheral edge region in the longitudinal direction of the second absorbing part to the first absorbing part. The compressed groove formed in the peripheral edge region in the longitudinal direction of the second absorbing part receives a stress from the body of the user and assists in taking a shape conforming to the shape of the body from the excretory part to the buttocks of the user, and thereby reducing leakage of the excreta during usage. On the other hand, the compressed groove formed to extend from the peripheral edge region in the longitudinal direction of the second absorbing part to the first absorbing part moderates the change of rigidity over the end part of the absorbent article from the first absorbing part to the second absorbing part, and assists in not only imparting rigidity to the first absorbing part and reducing twisting in the end part of the absorbent article but also reducing leakage of the excreta during usage.

The depth of the compressed groove formed in the peripheral edge region in the longitudinal direction of the second absorbing part is not particularly limited, and the compressed groove may be formed only within the second absorbing part to a depth insufficient to penetrate the first absorbing part, but as illustrated in Fig. 2, the compressed groove is preferably formed to a depth large enough to penetrate the second absorbing part and reach the first absorbing part. By virtue of the compressed groove reaching the first absorbing part, the rigidity in the end part of the absorbent article can be maintained and twisting can be reduced. On the other hand, in the case where the compressed groove is formed only within the second absorbing part, the position on the top surface of the first absorbing part, which corresponds to the compressed groove of the second absorbing part, is slightly recessed in correspondence with the groove, because the top surface of the first absorbing part is pressed by a pressure during embossing.

The shape of the compressed groove is a shape for forming a groove-like recess part on the absorbent article surface intended to abut on the skin. By continuously forming the compressed groove, lateral spreading of the excreta can be blocked and the excreta can be efficiently transferred to the lower side of the absorber.

In a preferred embodiment of the absorbent article of the present invention, the absorber for use in the present invention has an intermittent portion 8 where the compressed groove is not formed. The intermittent portion imparts a low rigidity portion to the absorber, and this is a portion working out to a folding line 11 when the absorbent article is housed in a package in a state of being folded in three. The position at which the intermittent portion is provided may be, in planar view, the peripheral end part in the longitudinal direction of the second absorbing part or may be the peripheral end part in the longitudinal direction of the first absorbing part. In the case where the intermittent part is provided within the second absorbing part, a low rigidity portion is formed at a predetermined position of the second absorber and in the case where the intermittent portion is provided within the first absorbing part, a low rigidity portion is formed at a predetermined position of the first absorber. Also, the intermittent portion may be provided in both the peripheral end part in the longitudinal direction of the first absorbing part and the peripheral end part in the longitudinal direction of the second absorbing part.

Fig. 3 illustrates a preferred embodiment of the present invention having an intermittent portion in the peripheral end part in the longitudinal direction of the second absorbing part. A pair of intermittent portions are provided in the peripheral end part in the longitudinal direction of the second absorbing part at a position inward by about 1 mm to about 40 mm from the edge of the second absorbing part. The distance in the intermittent portion is usually from about 2 mm to about 20 mm, preferably from about 3 mm to about 10 mm. If the distance in the intermittent portion is less than 2 mm, a low rigidity portion is not formed and the uncomfortable feeling during usage is disadvantageously not reduced, whereas if it exceeds 20 mm, the leakage prevention deteriorates and this is not preferred. As for the length of the compressed groove continuously provided in the peripheral edge region in the longitudinal direction of the second absorbing part, in the case where the absorbent article is, for example, a daytime sanitary napkin, the length in front and back of the center line in the longitudinal direction of the absorber is preferably about 15 mm or more from the standpoint of imparting deformability, more preferably about 25 mm or more from the standpoint of imparting stable deformability.

In the case where the intermittent portion is provided in the second absorbing part as illustrated in Fig. 3, the rigidity of the second absorbing part decreases and therefore, the absorbent article is allowed to hardly form a folding habit and readily restore the shape before folding when opening the package and can be easily fitted to the excretory part on which the absorbent article is abutted. In view of absorptivity, even when the intermittent portion is present in the second absorbing part, since a continuous compressed groove is present in the peripheral edge region in the longitudinal direction of the second absorbing part, leakage can be reduced by receiving a stress of the body and deforming the absorbent article upward. Also, by providing the intermittent portion in the second absorbing part, a larger second absorbing part can be designed and the absorption capacity can be increased.

On the other hand, in the case of providing the intermittent portion in the first absorbing part (not illustrated), a stress is concentrated at the portion with lowest rigidity out of the entire absorber, whereby the absorbent article can be easily coordinated to the action of the body. Also, even when the intermittent portion is present in the first absorbing part, a continuous compressed groove is present in the area from the peripheral end part in the longitudinal direction to the short direction of the first absorbing part and therefore, twisting can hardly occur. Furthermore, since the intermittent portion is not present in the second absorbing part, the size of the second absorbing part can be reduced while providing the required minimum absorption capacity and this reduction in size is advantageous in terms of packaging of the absorbent article in a state of being folded in three. In addition, by virtue of the absence of the intermittent portion in the second absorbing part that is abutted on the excretory part, uncomfortable feeling during usage can be reduced.

The above-described absorbers both can be preferably used in the absorbent article of the present invention, but in view of small reduction in the rigidity, an absorber having an intermittent portion in the second absorbing part is preferred.

In the conventional absorbent article, a groove part is formed only in the second pulp layer, making very high the rigidity of the second pulp part, and the package shape of the absorbent article is limited, because a folding line for folding and packaging the absorbent article is not present. On the other hand, in the present invention, as described above, the low rigidity portion works out to a folding line 11 when packaging the absorbent article, and the absorbent article of the present invention where the low rigidity portion can be formed at an arbitrary desired position is not limited in its package shape.

In the liquid-permeable sheet 7, a groove part having the same shape as the compressed groove in nearly closed form is formed at the same position as the compressed groove in closed form, and the liquid-permeable sheet 7 and the absorber 2 are integrated.

The absorber comprises at least a wood pulp but may be composed of a wood pulp with which a high-absorbent polymer is further mixed. Instead of a wood pulp, for example, a chemical pulp, a cellulose fiber and an artificial cellulose fiber such as rayon and acetate may be used. The absorbent polymer has a three-dimensional network structure in which a water-soluble polymer is appropriately crosslinked, and this polymer absorbs hundreds to thousands of times of water but is substantially water-insoluble and does not release the once absorbed water even when some pressure is applied. Examples thereof include starch-based, acrylic acid-based and amino acid-based particulate or fibrous polymers.

The absorbent article of the present invention can be used as a sanitary napkin or a sheet for urinary leakage.

The production method of an absorbent article of the present invention is described below. Specifically, the production method comprises the following steps (a) to (e) and is performed using a commonly employed production apparatus. The steps include (a) uniformly stacking pulp fibers on a paper mount 6 such as tissue by using a known fiber stacking apparatus for absorbent articles to form a first absorbing part 3 having almost the same dimension as the paper mount and having a nearly uniform thickness and a nearly uniform basis weight; (b) separately from the first absorbing part, forming a second absorbing part 4 smaller in the area than the first absorbing part in the same manner as in the formation of the first absorbing part and transferring the formed absorbing part onto the first absorbing part; and (c) wrapping the entire absorber with a paper mount and subjecting the absorber to compression and cutting into a predetermined shape.

The production method of the absorber 2 is not limited to those steps (a) and (b) as long as the absorber obtained takes a configuration where a first absorbing part 3 and a second absorbing part 4 are integrated. For example, an absorber in which a first absorbing part 3 and a second absorbing part 4 are integrated, may also be formed by a method where a support with the bottom having the same three-dimensional configuration as the configuration of a first absorbing part 3 and a second absorbing part 4 being integrated is disposed in the outer circumferential surface of a rotary drum of a fiber stacking apparatus and pulp fibers are then stacked on the support.

If desired, a nonwoven fabric or an air-laid pulp may be inserted as a second sheet between the liquid-permeable sheet 7 and the absorber 2. Also, an adhesion layer, for example, a layer comprising a hot-melt resin or a hot-melt adhesive, may be provided between the liquid-permeable sheet 7 and the absorber 2 or between the second sheet and the absorber 2.

Subsequently, (d) a step of embossing the absorber that is formed by cutting is performed. By the embossing, a compressed groove is provided. The embossing can be performed using a commonly employed apparatus. For example, embossing is applied by using an emboss roll having provided thereon a desired configuration of the compressed groove. In the case of providing a layer composed of a hot-melt resin or a hot-melt adhesive, the embossing is preferably heat embossing. By this embossing, a plurality of members constituting the absorbent article may be bound and integrated simultaneously with forming a compressed groove. For example, the permeable sheet 7 is supplied to the second absorbing part side of the absorber formed by cutting so as to cover the entire surface of the absorber and then embossed, whereby these can be integrated.

Simultaneously with embossing, a groove part 9 is sometimes formed also from the liquid-impermeable sheet 10 side, at the position corresponding to the compressed groove (see, Fig. 2). The groove part 9 is not formed directly by embossing but is formed indirectly by the reaction of embossing. The depth of the groove part 9 is usually very shallower than the depth of the compressed groove.

Finally, (e) a step of supplying a liquid-impermeable sheet 10 mainly comprising a polyethylene, a polypropylene or the like and joining the liquid-permeable sheet 7 and the liquid-impermeable sheet 10, is performed.

In the embodiment above, an example of the production method of a sanitary napkin is described, but the production method of the present invention can also be applied to production of other absorbent articles, for example, a sheet for urinary leakage.

## Claims

1. An absorbent article comprising at least a liquid-permeable sheet, a liquid-impermeable sheet, and an absorber sandwiched between the liquid-permeable sheet and the liquid-impermeable sheet,
wherein the absorber comprises a first absorbing part, and a second absorbing part formed nearly on the center part of the first absorbing part and the second absorbing part being smaller in the area than the first absorbing part, and
a compressed groove is continuously provided in nearly closed form to extend from the peripheral edge region in the longitudinal direction of the second absorbing part to the first absorbing part.

2. The absorbent article according to claim 1,
wherein the depth of said compressed groove continuously provided in the peripheral edge region in the longitudinal direction of the second absorbing part is a depth sufficient to penetrate the second absorbing part and reach the first absorbing part.

3. The absorbent article according to claim 1 or 2, wherein said absorber has an intermittent portion excluded from formation of the compressed groove, in the peripheral end part in the longitudinal direction of the second absorbing part.

4. The absorbent article according to claim 1 or 2, wherein said absorber has an intermittent portion excluded from formation of the compressed groove, in the peripheral end part in the longitudinal direction of the first absorbing part.

5. The absorbent article according to any one of claims 1 to 4, which is housed in a package in a state of being folded in three by making said intermittent portion work as a fold line.

6. A production method of an absorbent article, comprising stacking an absorber comprising a first absorbing part and a second absorbing part formed nearly on the center part of the first absorbing part and the second absorbing part being smaller in the area than the first absorbing part, a mount and a liquid-permeable sheet in this order on a mount, and continuously applying embossing from the liquid-permeable sheet side over a region from the peripheral edge region in the longitudinal direction of the second absorbing part to the first absorbing part, thereby providing a compressed groove extending from the second absorbing part to the first absorbing part.
